# EUROPEAN PATENT APPLICATION

(11) **EP 1 870 124 A1**
(43) Date of publication of application: **26.12.2007**
(21) Application number: 05788146.8
(22) Date of filing: 27.09.2005
(51) Int. Cl.: A61M 21/02, A61L 9/12

(54) **PERFUME COMPOUNDING METHOD AND PERFUME COMPOUNDING DEVICE**

(30) Priority: 02.12.2004 JP 2004350056; 02.12.2004 JP 2004350112
(71) Applicant: Mirapro Co., Ltd, Hokuto-city Yamanashi 408-0111 (JP)
(72) Inventor: ANDO, Takashi c/o MIRAPRO CO., LTD. 1100, 4080111 (JP); AKIYAMA, Shin c/o MIRAPRO CO., LTD. 1100, 4080111 (JP); SHOUGE, Naotaka c/o MIRAPRO CO., LTD. 1100, 4080111 (JP); SHIMIZU, Tetsuya c/o MIRAPRO CO., LTD. 1100, 4080111 (JP)
(74) Representative: TBK-Patent
(86) International application number: PCT/JP2005/017710
(87) International publication number: WO 2006/059426

(57) **Abstract**

An aroma dispensing method and an aroma dispensing device are provided which do not require a complicated controlling method or a large-scaled system, which employs a novel smell dispensing mode, and which are cost-saving and have good usability. The aroma dispensing device comprises: an air pump 2 for supplying air; six element smell bottles 5a-5f for containing smell materials and for holding smells generated by blowing the air against the smell materials; and six solenoid valves for controlling of the air to be introduced into the element smell bottles; wherein when the plurality of smells generated in the six element smell bottles are blended in specific mixing proportions, the mixing proportions are determined by numbers or time periods of opening and closing per second of the six solenoid valves respectively connected to the six element smell bottles; and proportions of the numbers or time periods of opening and closing per second of the solenoid valves correspond to the mixing proportions.

## Description

### [Technical Field]

The present invention relates to an aroma dispensing method and an aroma dispensing device. More particularly, the present invention relates to an aroma dispensing method and an aroma dispensing device which allow a person to privately enjoy a specific aroma in a room for healing or relaxation or which generate a specific smell for deodorizing an unpleasant smell such as a smell of cigarette in a room.

### [Background Art]

In daily life in which we are likely to be under stress, a role which a smell or aroma plays is greater than we think. For example, a deodorant is placed in a rest room, and a good aroma is allowed to emanate from the deodorant in order to deodorize an unpleasant smell. Further, in a closed space, an odor is likely to prevail due to poor ventilation to cause unpleasantness. However, when a deodorant is placed or sweet-smelling flowers are placed in the space, the unpleasant odor is thereby removed. In particular, an aromatic is often placed in an automobile to remove an unpleasant smell in the closed automobile.

Moreover, if we have a good smell such as a scent of rose sitting on a sofa when tired with work, we feel refreshed. In addition, if a child has, in conformity with fruit drawn in a picture book for children, the actual smell of the fruit, understanding and recognition of the child are increased. With respect also to an adult, if the adult, while watching images of a TV program or video, actually has a smell or aroma which matches with the images, realism is greatly enhanced.

As described above, effect and function which a smell or aroma has are great. Accordingly, in recent years, developments of techniques for recording and reproducing a smell have been made actively. In smell, however, no counterparts of the three primary colors in light or color have yet been defined. As matters now stand, the developments have been advanced independently in each of research institutes. In other words, there are no standards on a plurality of basic element smells, and with respect also to recipes for specific smells, there are no references, standards, definitions or the like. Accordingly, these are independently prescribed by each of the research institutes. In consequence, although smells can be reproduced based on recipes stored in the form of data in one research institute, recipes are not standardized among research institutes. Accordingly, the recipes stored in the form of data cannot be used in common.

Fig.7 shows a dispensing device disclosed in Patent Document 1. A case where one-component smell is dispensed is shown. The dispensing device comprises, as main components, a carrier gas bottle 17, a sample bottle 18, an element smell gas bottle 19, solenoid valves for switching 28 and 29, a sensor cell 30, a pump 31, a frequency counter 32, and a computer 33.

The dispensing device determines a smell in the sample bottle 18 and records data on the smell in the computer and reproduces (dispenses) the smell in the sample bottle 18 based on the recorded data on the smell.

The determination system is a gas flow determination system which supplies either of a carrier gas or a sample smell to the sensor 30 at a predetermined flow rate. To supply the gas to the sensor 30 at the predetermined flow rate, the gas flow is controlled using the solenoid valve 28. The solenoid valve 28 is capable of controlling the flow rate in response to electrical signals from outside, and the flow rate control is performed by the electrical signals from the computer 33. The solenoid valve 29 is used to permit the carrier gas to flow, thereby diluting the sample smell or a dispensed smell. Although response of the sensor 30 is relatively rapid, its recovery time is long with respect to high concentration gases. Accordingly, the system is so constructed that with respect to high concentration gases, they are diluted and then supplied to sensors 30A and 30B.

A solenoid valve 27 is for supplying the sample smell. By controlling the solenoid valves 28, 29 to regulate flow rate proportions, a concentration of an element smell gas can be changed. The solenoid valves are used for the flow control. The solenoid valves 27, 28 and 29 are operated complementarily to switch between the carrier gas, the sample smell, and the dispensed smell. The sensor cell 30 is for housing a sensor 30A and a sensor 30B therein. In the cell, two quartz crystal microbalance gas sensors 30A, 30B having different characteristics are disposed. Downstream from the sensor cell 30, a pump 31 is provided via a valved flowmeter 40 to drive gas flow.

It is necessary to keep a headspace of each of the carrier gas bottle 17, the sample bottle 18 and the element smell gas bottle 19 and a flow rate in the sensor cell 30 constant irrespective of the switchover of the solenoid valves. Accordingly, even when no smell is supplied to the sensor 30, air is supplied to the headspace in each of the bottles through other bypass channels. The system has a symmetric structure so that gases flow through the bottles and the air system at equal flow rates. Accordingly, the air system is also provided with the carrier gas bottle 17 (empty sample bottle).

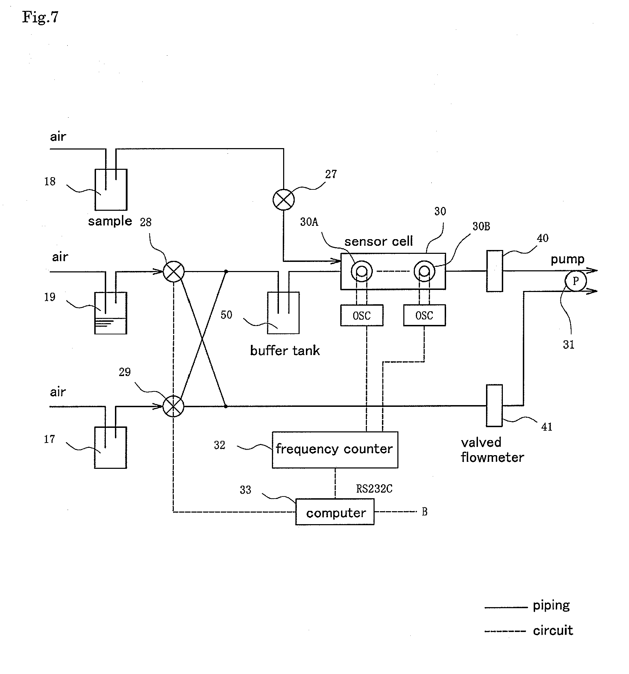

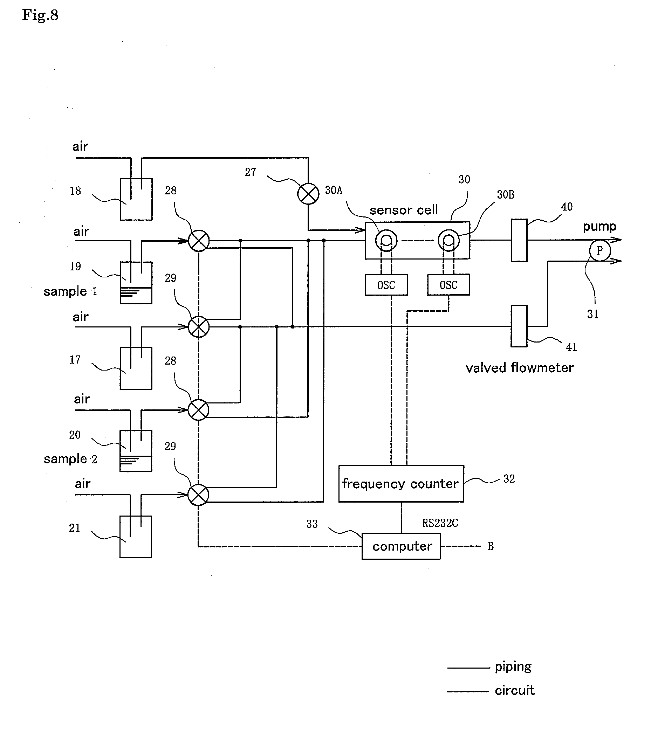

Fig.8 shows a dispensing device which dispenses a two-component smell. Since this device has the same function as in the device in Fig.7 except that an element smell bottle 20 and a carrier gas bottle 21 are added, explanation thereon is omitted.

The dispensing devices disclosed in Patent Document 1 have the above-described structures and are characterized in the following points.
(1) The recipe is determined based on the mixing ratio of the element smell or smells. The final mixing ratio is determined by sequentially performing pattern matching while changing the mixing ratio of the element smell or smells.
(2) Opening and closing of the solenoid valves are performed by Δ - Σ modulation method.
(3) Each element smell and the carrier gas are paired.
(4) The mixing ratio of the element smell or smells can be converted into an electrical signal.
(5) The mixing ratio of the element smell or smells can be recorded.
(6) The mixing ratio of the element smell or smells can be transmitted via a communication medium.
(7) A smell can be reproduced based on the recorded mixing ration of the element smell or smells.

According to the dispensing devices disclosed in Patent Document 1, a recipe for an object gas ca be obtained with high accuracy, and a smell of the object gas can be reproduced with high accuracy by means of the element smell gas or gasses. However, the devices are considered to have the following problems.
(1) To determine the recipe for the object smell, such a method is employed that the pattern matching is performed on the basis of measurement results from the sensor array, and the pattern matching is sequentially improved while changing the mixing ratio of the element smell or smells. Accordingly, the complicated device and a lot of time are required to determine the recipe for the object smell gas.
(2) The recipe is the mixing ratio of the element smell or smells. Accordingly, it is necessary to precisely determine a concentration of each element smell, and thus the data on the recipe is complex.
(3) Since switching of the solenoid valves is performed using the Δ - Σ modulation method, control of the switching is complicated.
(4) The devices have such a structure that the smells remain on the solenoid valves. It is, therefore, necessary to periodically deodorize the solenoid valves.
(5) Each element smell gas and the carrier gas are paired, and the flow rate in the sensor cell is required to be constant irrespective of the switchover of the solenoid valves. Accordingly, the devices have such a structure that use efficiency of the carrier gas is poor.

Patent Document 1:
Japanese Unexamined Patent Publication No.2002-277367

### [Disclosure of Invention]

### [Problems to be Solved by the Invention]

As described above, many problems remain unsolved in research on smells or aromas. The dispensing devices disclosed in Patent Document 1 are considered to have many problems in terms of usability.

The present invention has been made in view of the problems inherent in the conventional techniques. It is, therefore, an object of the present invention to provide an aroma dispensing method and aroma dispensing device which do not require a complicated controlling method or a large-scaled system, and which are cost-saving and have good usability.

### [Means to Solve the Problems]

One mode of the aroma dispensing method of the present invention comprises:
blowing air against a plurality of smell materials contained in a plurality of element smell bottles to volatilize the plurality of smell materials, there by generating a plurality of smells; and
blending the plurality of generated smells in specific mixing proportions;
wherein the mixing proportions are determined by numbers of opening per second of a plurality of solenoid valves (numbers of open condition per second of the solenoid valves: hereinafter referred to as "numbers of opening and closing per second of the plurality of solenoid valves") which are respectively connected to the element smell bottles; and
proportions of the numbers of opening and closing per second of the plurality of solenoid valves correspond to the mixing proportions.

Each of the element smell bottles may be a glass bottle or a container made of a metal. In each of the element smell bottles, a smell material is contained. The smell material may be a liquid or solid. By blowing air against the smell material, the smell material is volatilized to generate a smell.

Each of the solenoid valve is electrically switched on and off to control air flow. In general, the solenoid valve has one inlet and two outlets. When the solenoid valve is ON or open, air introduced from the inlet is released from one outlet, and when the solenoid valve is OFF or closed, the air is released from the other outlet. Opening and closing of the solenoid valve can be controlled by pulse operation in which one second is divided into a plurality of intervals taking a time required for one cycle motion into consideration. For example, when one operation interval is supposed to be 50 msec, pulse controlling in which one second is divided into 20 time periods can be performed. In this case, to keep the solenoid valve open, 20 pulses are applied valve per second. In this case, the amount of air introduced into the element smell bottle is the maximum. When half of the maximum air amount is introduced into the element smell bottle, 10 pulses are applied per second. In other words, a number of opening and closing of the solenoid valve is 10 per second. In this manner, by the number of opening and closing per second of the solenoid valve, the amount of air introduced into the element smell bottle can be controlled.

A solenoid valve is allotted to each of the element smell bottles, and the solenoid valve performs the above-described pulse operation. Accordingly, in a case where a number of the element smell bottles is supposed to be n and smells generated from the n element smell bottles are blended, mixing proportions of the smells can be controlled by the number of pulses or number of opening and closing per second.

For example, when n = 4 and smells are blended in proportions of 50% : 20% : 15% : 15%, numbers of opening and closing per second of the solenoid valves are 10 times : 4 times : 3 times : 3 times, wherein an operation interval of the solenoid valves is 50 msec. In this manner, the proportions of blended smells can be controlled accurately and linearly by the numbers of opening and closing per second of the solenoid valves.

Therefore, according to the one mode of the aroma dispensing method of the present invention, in blending of the smells generated from the plurality of element smell bottles, the mixing proportions can be controlled accurately by the numbers of opening and closing per second of the solenoid valves which control amounts of air to be introduced into the element smell bottles. The proportions of the blended smells can be regulated linearly.

Another mode of the aroma dispensing method of the present invention comprises:
blowing air against a plurality of smell materials contained in a plurality of element smell bottles to volatilize the plurality of smell materials, there by generating a plurality of smells; and
blending the plurality of generated smells in specific mixing proportions;
wherein the mixing proportions are determined by (total) time periods of opening per second of a plurality of solenoid valves ((total) time periods of open condition per second of the solenoid valves: hereinafter referred to as "time periods of opening and closing per second of the plurality of solenoid valves") which are respectively connected to the element smell bottles; and
proportions of the time periods of opening and closing per second of the plurality of solenoid valves correspond to the mixing proportions.

Operation of the solenoid valves is as described above. For example, when a number of the element smell bottoms n = 4 and smells are blended in mixing proportions of 50% : 20% : 15% : 15%, numbers of opening and closing per second of the solenoid valves are 10 times : 4 times : 3 times : 3 times, wherein an operation interval of the solenoid valves is 50 msec. When these numbers of opening and closing per second of the solenoid valves are converted into time periods of opening and closing per second of the solenoid valves, the time periods are 500 msec, 200 msec, 150 msec and 150 msec, respectively. In other words, proportions of the time periods of opening and closing per second of the solenoid valves are 500 : 200 : 150 : 150 = 10 : 4 : 3 : 3 and equal to the mixing proportions of the smells.

Therefore, according to the another mode of the aroma dispensing method of the present invention, in the blending of the smells generated from the plurality of element smell bottles, the mixing proportions can be controlled accurately by the time periods of opening and closing per second of the solenoid valves which control amounts of air to be introduced into the element smell bottles. The proportions of the blended smells can be regulated linearly.

Still another mode of the aroma dispensing method of the present invention comprises:
blowing air against a plurality of smell materials contained in a plurality of element smell bottles to volatilize the plurality of smell materials, there by generating a plurality of smells; and
blending the plurality of generated smells in specific mixing proportions;
wherein the mixing proportions are determined by a plurality of solenoid valves respectively connected to inlets of the plurality of element smell bottles; and
the air is sent to the element smell bottles when the solenoid valves are open, and the air is returned to an air supplying chamber for temporarily holding air which is connected to the solenoid valves when the solenoid valves are closed.

The air supplying chamber is a chamber for temporarily holding air supplied from an air pump and distributing the air among the solenoid valves, and a size of the air supplying chamber may appropriately be determined. The air supplying chamber may be made of an arbitrary material. The air supplying chamber is provided with only one inlet, and the air delivered from the air pump is introduced from the inlet and evenly discharged from outlets which are provided in the same number as that of the element smell bottles. The outlets are respectively connected to the solenoid valves, and since this aroma dispensing method is constructed such that the air is returned to the air supplying chamber when the solenoid valves are OFF or closed, the air supplying chamber is provided with return ports in the same number as that of the solenoid valves.

Therefore, according to the still another mode of the aroma dispensing method, the air is returned to the air supplying chamber when the solenoid valves are OFF. Accordingly, the air supplied from the air pump can be used efficiently without wasting the air.

In the one or another mode of the aroma dispensing method of present invention, it is preferred that the numbers or time periods of opening and closing per second of the plurality of solenoid valves be modified according to temperatures measured by a temperature measuring means disposed in an aroma dispensing device.

The smell materials as sources of smells are volatilized in temperature-dependent amounts. Accordingly, it is desirable that the numbers or time periods of opening and closing per second of the solenoid valves be modified according to temperatures. The modification can be effected by increasing or reducing the numbers or time periods of opening and closing according to temperatures. Degrees of the increase or reduction may appropriately be determined according to types of the smell materials used or the like. Therefore, according to this mode of the aroma dispensing method, a dispensed smell is not changed even if the operating temperature of the aroma dispensing device is changed. This increases reliability of the generated smell.

One form of the aroma dispensing device of the present invention comprises:
a plurality of element smell bottles for containing smell materials and for holding smells generated by blowing air against the smell materials;
an air pump for supplying the air;
an air supplying chamber for temporarily holding the air supplied by the air pump;
a plurality of solenoid valves for controlling flow rates of the air in introduction of the air from the air supplying chamber to the plurality of element smell bottles; and
a blender chamber to which the air controlled by the plurality of solenoid valves is introduced for blending the smells generated in the plurality of element smell bottles;
wherein when the plurality of smells generated in the plurality of element smell bottles are blended in the blender chamber in specific mixing proportions, the mixing proportions are determined by numbers of opening and closing per second of the plurality of solenoid valves respectively connected to the plurality of element smell bottles; and
proportions of the numbers of opening and closing per second of the plurality of solenoid valves correspond to the mixing proportions.

The element smell bottles and the solenoid valves are as described above. The air pump is for supplying air, and an amount of air delivered per second may appropriately be determined. The air supplying chamber is a chamber for temporarily holding air supplied from an air pump and distributing the air among the solenoid valves, as described above, and a size of the air supplying chamber or the like may appropriately be determined. The air supplying chamber may be made of an arbitrary material.

The blender chamber is a chamber for blending smells generated in the element smell bottles and releasing the dispensed smell, and the blender chamber is so constructed that the smell dispensed therein is released from the top outlet portion thereof. Accordingly, the outlet portion of the blender chamber preferably has a shape which allows the dispensed smell to easily pass through or leave. For this purpose, the blender chamber may have such a tapered sectional shape that its top outlet portion upward expands. The blender chamber is provided with inlets for introducing smell-containing airs from the respective solenoid valves in the same number as that of the solenoid valves, and positions of the inlets may appropriately be determined. For example, the inlets may be provided in the bottom or may be circumferentially provided near the middle of the blender chamber. As connecting means between the air pump and the air supplying chamber, the air supplying chamber and the solenoid valves, the solenoid valves and the element smell bottles, and the element smell bottles and the blender chamber, vinyl pipes or the like may be used. It is preferred to select a material on which no substantial smells remain.

A solenoid valve is allotted to each of the element smell bottles, and the solenoid valve performs the above-described pulse operation. Accordingly, in a case where a number of the element smell bottles is supposed to be n, and smells generated from the n element smell bottles are blended, mixing proportions of the smells can be controlled by the number of pulses or number of opening and closing per second of the solenoid valve.

Therefore, according to the one form of the aroma dispensing device of the present invention, in the blending of the smells generated from the plurality of element smell bottles, the mixing proportions can be controlled accurately by the numbers of opening and closing per second of the solenoid valves which control amounts of air to be introduced into the element smell bottles. The proportions of the blended smells can be regulated linearly.

Another form of the aroma dispensing device of the present invention comprises:
a plurality of element smell bottles for containing smell materials and for holding smells generated by blowing air against the smell materials;
an air pump for supplying the air;
an air supplying chamber for temporarily holding the air supplied by the air pump;
a plurality of solenoid valves for controlling flow rates of the air in introduction of the air from the air supplying chamber to the plurality of element smell bottles; and
a blender chamber to which the air controlled by the plurality of solenoid valves is introduced for blending the smells generated in the plurality of element smell bottles;
wherein when the plurality of smells generated in the plurality of element smell bottles are blended in the blender chamber in specific mixing proportions, the mixing proportions are determined by time periods of opening and closing per second of the plurality of solenoid valves respectively connected to the plurality of element smell bottles; and
proportions of the time periods of opening and closing per second of the plurality of solenoid valves correspond to the mixing proportions.

Operation of the solenoid valves is as described above. For example, when a number of the element smell bottoms n = 4 and smells are blended in mixing proportions of 50% : 20% : 15% : 15%, numbers of opening and closing per second of the solenoid valves are 10 times : 4 times : 3 times : 3 times, wherein an operation interval of the solenoid valves is 50 msec. When these numbers of opening and closing per second of the solenoid valves are converted into time periods of opening and closing per second of the solenoid valves, the time periods are 500 msec, 200 msec, 150 msec and 150 msec, respectively. In other words, proportions of the time periods of opening and closing per second of the solenoid valves are 500 : 200 : 150 : 150 = 10 : 4 : 3 : 3 and equal to the mixing proportions of the smells.

Therefore, according to the another form of the aroma dispensing device of the present invention, in the blending of the smells generated from the plurality of element smell bottles the mixing proportions can be controlled accurately by the time periods of opening and closing per second of the solenoid valves which control amounts of air to be introduced into the element smell bottles. The proportions of the blended smells can be regulated linearly.

Still another form of the aroma dispensing device of the present invention comprises:
a plurality of element smell bottles for containing smell materials and for holding smells generated by blowing air against the smell materials;
an air pump for supplying the air;
an air supplying chamber for temporarily holding the air supplied by the air pump;
a plurality of solenoid valves for controlling flow rates of the air in introduction of the air from the air supplying chamber to the plurality of element smell bottles; and
a blender chamber to which the air controlled by the plurality of solenoid valves is introduced for blending the smells generated in the plurality of element smell bottles;
wherein when the plurality of smells generated in the plurality of element smell bottles are blended in the blender chamber in specific mixing proportions, the mixing proportions are determined by the plurality of solenoid valves respectively connected to inlets of the plurality of element smell bottles; and
the air is sent to the element smell bottles when the solenoid valves are open, and the air is returned to the air supplying chamber for temporarily holding air which is connected to the solenoid valves when the solenoid valves are closed.

The air supplying chamber is a chamber for temporarily holding air supplied from the air pump and distributing the air among the solenoid valves, and a size of the air supplying chamber or the like may appropriately be determined. The air supplying chamber may be made of an arbitrary material. The air supplying chamber is provided with only one inlet, and the air delivered from the air pump is introduced from the inlet and evenly discharged from outlets which are provided in the same number as that of the element smell bottles. The outlets are connected to the solenoid valves, and since this aroma dispensing device is so constructed that the air is returned to the air supplying chamber when the solenoid valves are OFF or closed, the air supplying chamber is provided with return ports in the same number as that of the solenoid valves.

Therefore, according to the still another form of the aroma dispensing device, the air is returned to the air supplying chamber when the solenoid valves are off. Accordingly, the air supplied from the air pump can be used efficiently without wasting the air.

In the one or another form of the aroma dispensing device, it is preferred that the numbers or time periods of opening and closing per second of the plurality of solenoid valves be modified according to temperatures measured by a temperature measuring means disposed in the aroma dispensing device, as a further form of the aroma dispensing device.

The smell materials as smell sources are volatilized in temperature-dependent amounts. Accordingly, it is desirable that the numbers or time periods of opening and closing per second of the solenoid valves be modified according to temperatures. The modification can be effected by increasing or reducing the numbers or time periods of opening and closing according to temperatures. Degrees of the increase or reduction may appropriately be determined according to types of the smell materials used or the like. Therefore, according to the further form of the aroma dispensing device, a dispensed smell is not changed even if the operating temperature of the aroma dispensing device is changed. This increases reliability of the generated smell.

In any of the one to further forms of the aroma dispensing device, it is preferred that the plurality of element smell bottles be bar-coded and managed with the bar codes; and that the aroma dispensing device have an arithmetic element for calculating smells which can be dispensed by means of the plurality of element smell bottles and a database for storing the results of the calculation by the arithmetic element, as a still further form of the aroma dispensing device.

Information on each of the element smell bottles can be read by means of the bar-code applied to the element smell bottle. The arithmetic element is for calculating smells which can be blended by means of the plurality of element smell bottles contained in the aroma dispensing device based on the information read from the bar codes, and the results of the calculation can be recoded as recipes for the smells. The database is for recording the results of the calculation, and a general-purpose recording medium or the like can be used therefor.

Therefore, according to the still further form of the aroma dispensing device, the element smell bottles are managed with the bar codes, and the recipes for the smells which can be blended are stored in the database. Accordingly, it is possible to produce a desired smell with ease.

In any of the one to still further forms of the aroma dispensing device, it is preferred that the numbers or time periods of opening and closing per second of the plurality of solenoid valves be converted into electrical signals and thereby can be delivered over internet, as another preferred form of the aroma dispensing device..

With respect to each of the element smell bottles, a reference number of the element smell bottle and the number or time period of opening and closing per second of the solenoid valve which are managed by means of the bar code correspond to the recipe for the smell, and the recipe is converted into an electrical signal. The smell can be delivered via internet by transmitting the electrical signal to an aroma dispensing device as a receiver via internet. However, if types of element smell bottles are different between the aroma dispensing device as a transmitter and the aroma dispensing device as a receiver, it is possible that the transmitter transmits a recipe, and the receiver specifies a smell from the received recipe to check, with reference to the database, whether or not the smell can be dispensed by means of the element smell bottles which the receiver has. If no information on the same smell is stored in the database, the receiver may be so adapted as to generate a similar smell.

Therefore, according to the another preferred form of the aroma dispensing, a recipe of a smell can be delivered via internet. Accordingly, it is possible to enjoy the same smell or aroma at any places in the world.

In any of the one to another preferred forms of the aroma dispensing device, it is preferred that when the solenoid valves are open to generate a smell, a light emitting diode be caused to glow so as to effect cooperation between the smell and light of the light emitting diode, as still another preferred form of the aroma dispensing device.

As the light emitting diode, an ordinary LED can be used. Light with a specific color may be emitted or colors of light may randomly be changed when a smell is generated. In this manner, it is possible to enjoy cooperation of the smell with the color or colors.

In any of the one to still another preferred forms of the aroma dispensing device, it is preferred that when the solenoid valves are open to generate a smell, a sound be made so as to effect cooperation between the smell and the sound.

A sound or music may be generated or a music may be played from a built-in speaker of the aroma dispensing device. A sound is generated or a music is played when a smell is generated to thereby enjoy cooperation between the smell and the sound.

### [Effect of the Invention]

According to the aroma dispensing method and the aroma dispensing device of the present invention, in blending of smells generated from a plurality of element smell bottles, mixing proportions of the smells can be controlled accurately by numbers or time periods of opening and closing per second of solenoid valves which introduces air into the element smell bottles. The mixing proportions can be changed linearly. As a result, it is possible to provide an aroma dispensing method and aroma dispensing device which do not require a complicated controlling method or a large-scaled system, and which are cost-saving and have good usability.

### [Best Mode for Carrying Out the Invention]

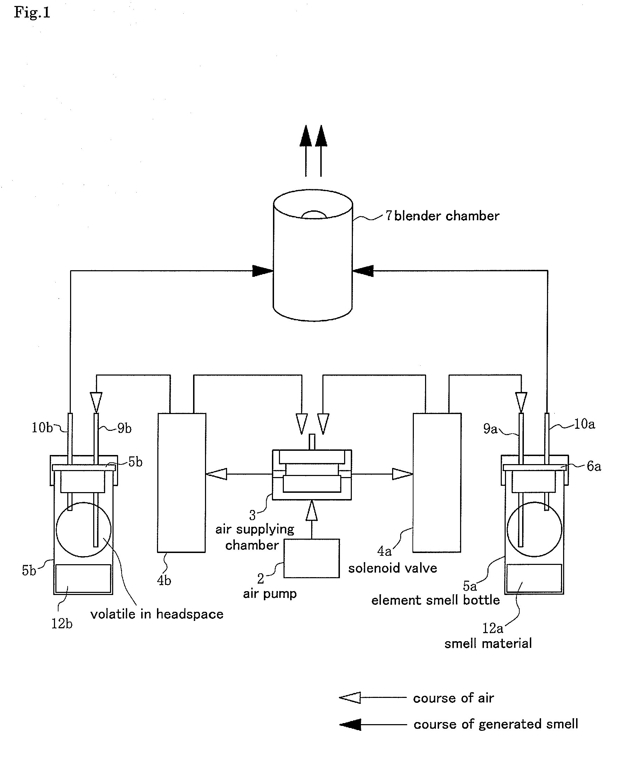

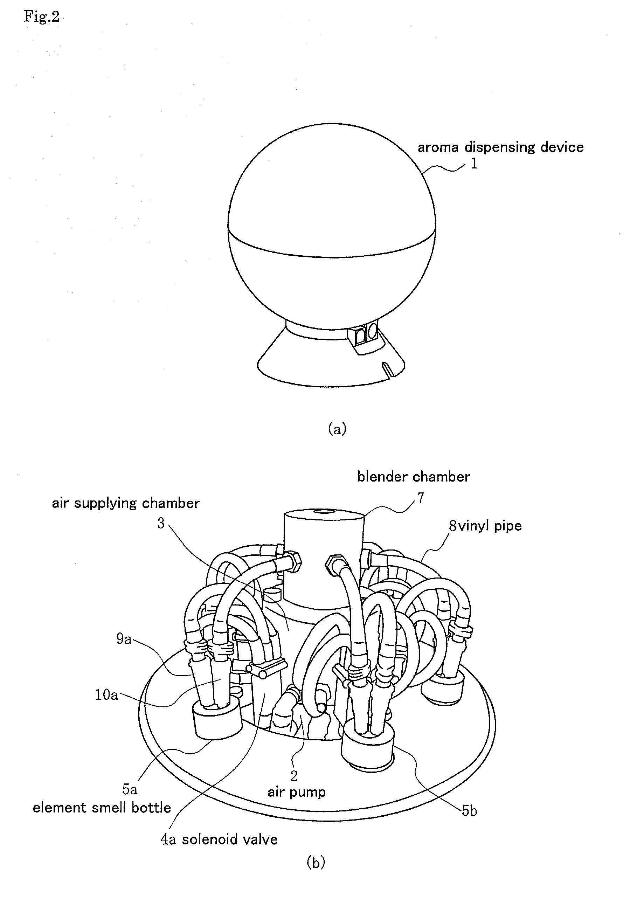

Hereinafter, the aroma dispensing method and the aroma dispensing device of the present invention will be described in detail with reference to the drawings. Fig.1 is a schematic diagram of a first embodiment of the aroma dispensing device according to the present invention. Figs.2(a) and 2(b) are a perspective overall view and a perspective view of an operative portion, respectively, of the first embodiment of the aroma dispensing device according to the present invention.

The aroma dispensing device 1 of the first embodiment comprises an air pump 2, an air supplying chamber 3, six element smell bottles, six solenoid valves, and a blender chamber 7. The six element smell bottles are a first element smell bottle 5a, a second element smell bottle 5b, a third element smell bottle 5c, a fourth element smell bottle 5d, a fifth element smell bottle 5e, and a sixth element smell bottle 5f. The six solenoid valves are a first solenoid valve 4a, a second solenoid valve 4b, a third solenoid valve 4c, a fourth solenoid valve 4d, a fifth solenoid valve 4e, and a sixth solenoid valve 4f. Besides these, the device is provided with a thermistor for measuring a temperature, an LED which glows when the air pump is operated to generate a smell, a speaker which makes a sound also when a smell is generated, a control board for controlling the speaker and the LED, a control board for controlling the solenoid valves, an interface for connecting the device to a computer for internet delivery of smell, and the like, although they are not shown. In this connection, although only two element smell bottles and two solenoid valves are shown in Fig.1, the six element smell bottoms and the six solenoid valves are provided in reality.

The element smell bottles 5a-5f have a cylindrical shape each and can contain smell materials 12a-12f as smell sources in bottom portions thereof, respectively. The element smell bottles 5a-5f are provided with element smell bottle lids 6a-6f at their tops, respectively. Through the element smell bottom lids 6a-6f, inlet pipes 9a-9f for introducing air delivered from the solenoid valves 4a-4f and outlet pipes 10a-10f for delivering smell-containing air are provided, respectively. The inlet pipes 9a-9f are connected to the solenoid valves 4a-4f via vinyl pipes 8, respectively. Each of the outlet pipes 10a-10f are connected to the blender chamber 7 via a vinyl pipe 8. Each of the element smell bottles 5a-5f, the element smell bottle lids 6a-6f, the inlet pipes 9a-9f, and the outlet pipes 10a-10f is made of a plastic resin.

The inlet pipes 9a-9f for introducing air which are provided through the element smell bottle lids 6a-6f, respectively, are inserted to vicinities of the surfaces of the smell materials 12a-12f as smell sources, respectively. Air which is supplied by the air pump 2 and introduced from the inlet pipes 9a-9f via the solenoid valves 4a-4f is blown against the surfaces of the smell materials 12a-12f as smell sources to volatilize the smell materials 12a-12f, thereby generating smells. The plurality of generated smells are mixed to obtain a desired smell. As described below, in some cases, when air is introduced in a larger amount, a smell is generated in a larger amount to result in a higher concentration of the smell. In some other cases, depending upon a smell material used, if air is introduced even in a larger amount, an amount of a volatilized component is not changed and thus a concentration of a smell is conversely lowered. It is, accordingly, desired that a total amount a smell-containing air released from the blender chamber 7 be preliminarily determined and, based on the total amount, amounts of air introduced into the element smell bottles be determined. It should be noted that the smell materials 12a-12f may be liquid or solid.

The outlet pipes 10a-10f for leading the smells generated in the element smell bottles 5a-5f to the blender chamber 7, which are provided through the element smell bottle lids 6a-6f, are lightly inserted to vicinities of the lower surfaces of the element smell bottle lids 6a-6f, respectively, and not deeply inserted into the element smell bottles in order to enable smells generated between the element smell bottle lids 6a-6f and the upper surfaces of the smell materials 12a-12f as smell sources to be efficiently taken. In Fig.1, such spaces, i.e., portions in which volatilized components are present in the element smell bottles are shown by circular marks as the volatilized components in the headspaces.

The air pump 2 is for supplying air to the air supplying chamber 3, and a simple general-purpose one is used as the air pump. A total amount of air pumped in a unit time is appropriately determined by a size of the air supplying chamber 3 used, a number of the solenoid valves, an inner diameter of the vinyl pipes 8, a size of the blender chamber 7 and the like. Since noise of the air pump will make a problem, one with a minimum of noise is preferably used.

The air supplying chamber 3 plays a role to temporarily hold air sent from the air pump 2 and then distribute the air to the first to sixth solenoid valves. In other words, the air supplying chamber 3 is provided with one inlet connected to the air pump 2 via the vinyl pipe 8 and six outlets connected to the first to sixth solenoid valves 4a-4f via vinyl pipes 8, and air introduced through the inlet is temporarily held in the air supplying chamber 3 and the held air is discharged evenly from the six outlets. Further, as described blow, since the air discharged from the air supplying chamber 3 is returned thereto and reused when the solenoid valves 4a-4f are OFF or closed, the air supplying chamber 3 is provided with six return ports therefor.

The air discharged from the outlets of the air supplying chamber 3 is introduced into the six solenoid valves 4a-4f. In each of the solenoid valves 4a-4f, the valve is switched on-off by a solenoid to control air flow. In this embodiment, EXAK-3 which is a three-way valve in EXAK series of Takasago Electric Inc. is used as each of the solenoid valves. The three-way valve is of a type which has one inlet and two outlets. Although operation of the solenoid valves for blending smells will be described in detail below, the air introduced from the inlet is discharged from one of the outlets and sent to the element smell bottle when the solenoid valve is ON or open. The air is discharged from the other outlet and returned to the air supplying chamber 3 when the solenoid valve is OFF or closed.

The air returned when the solenoid valve or valves are closed enters the air supplying chamber 3 and is re-send to the solenoid valve or valves. Accordingly, after the air supplied from the air pump 2 is temporarily pooled in the air supplying chamber 3, the air is not discharged from the air supplying chamber 3 without being permitted to return thereto in the case other than that where the air is used for generating a smell. The air supplied from the air pump 2 is used efficiently.

The blender chamber 7 has six inlets, and the six inlets are connected to the outlet pipes 10a-10f of the element smell bottle lids 6a-6f via the vinyl pipes 8, respectively. The pairs of pipes extending from the element smell bottle lids 6a-6f, i.e., the inlet pipes 9a-9f and the outlet pipes 10a-10f have the same respective lengths. The blender chamber 7 has a cylindrical shape and is open-topped, and a smell is released from the opening. The inlets of the blender chamber 7 are circumferentially provided near the middle of the blender chamber 7, and six smells are introduced from the inlets and blended in the blender chamber 7, and the resulting desired smell or aroma is released from the top opening. In consequence, the smell prevails in the entire room in which the aroma dispensing device 1 is placed.

The blender chamber 7 preferably has a sectional structure which allows the dispensed smell to easily leave. In the present invention, the blender chamber 7 is provided with a downward tapered opening portion at its top. The tapered portion has such a shape that the opening portion upward expands. The smell is thereby efficiently emitted from the blender chamber 7, and the desired smell or aroma floats in a room. The blender chamber 7 is made of Teflon (registered trademark) in view of easiness of processing and taking it into consideration that the blender chamber is detached and washed.

In the aroma dispensing device 1 of the present invention, the solenoid valves 4a-4f are located upstream from the element smell bottles 5a-5f, respectively. By virtue of this structure, no smells remain on the solenoid valves 4a-4f. Accordingly, there is no troublesomeness inherent in the conventional techniques that residual smells remaining on solenoid valves should be deodorized every time when a smell to be generated is changed. Thus, a desired smell can be generated in a short period of time.

The total amount of the air supplied to the element smell bottles 5a-5f is equal to the total amount of the air discharged from the element smell bottles 5a-5f, and this amount of the air flows into the blender chamber 7. This amount is defined as a flow volume. The flow volume V is given as V = a cross-sectional area of a smell supplying pipe (S) x a pressure in the pipe (P) x a solenoid valve opening time (T), wherein the cross-sectional area of a smell supplying pipe is an inner cross-sectional area of the vinyl pipe, and the pressure in the pipe is a pressure in the vinyl pipe. If S and P are constant, V is determined depending upon the solenoid valve opening time T.

In the next place, operation of the solenoid valves will be described. Opening and closing of the solenoid valve are performed by pulse operation at 20Hz. An operation interval of the pulse is 50 msec. In other words, the opening and closing of the solenoid valve is controlled by dividing one second into 20 sections.

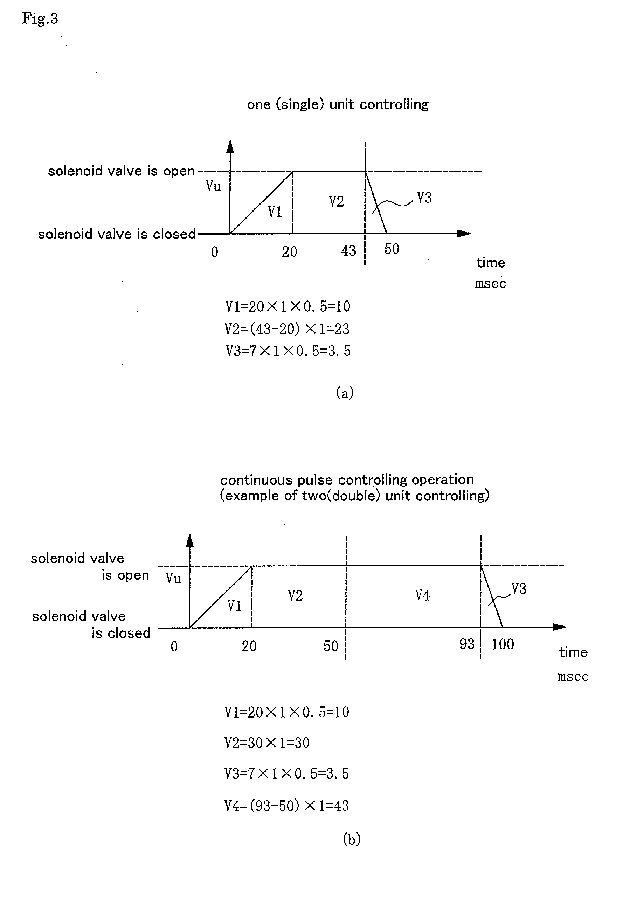

Here, one unit controlling operation is defined as a solenoid valve opening operation by one pulse when the opening and closing are controlled at 20Hz. Fig.3 is illustrative views on one unit controlling operation (single unit controlling operation) Fig.3(a) and continuous pulse controlling (case of double unit controlling) operation Fig.3(b). The vertical axis represents degree of opening (full open state is defined as 1), and the horizontal axis represents time (unit: msec).

A unit of the flow volume V in which a smell-containing gas (for example, a smell-containing air) passes through the solenoid valve when the solenoid valve is ON or open is defined as Vu·msec. In this connection, Vu is a unit volume and defined herein as 1. In other words, when the solenoid valve is in a full-open state, i.e., the unit is 1msec, V=1. In the following, flow volumes will be calculated on the two cases shown in Fig.3. In the case of one (single) unit controlling, the flow volume V1 is V1 = 20 x 1/2 + (43 - 20) x 1 + (50 -43) x 1/2 = 10 + 23 + 3.5 = 36.5. In the case of two (double) unit controlling, the flow volume V2 is calculated in the same manner, and V2 = 10 + 30 + 43 + 3.5 = 86.5. A flow volume Vn in n-continuous pulse controlling operation is expressed as Vn = 36.5 + 50 x (n-1) wherein n is a unit pulse number (or simply, a unit).

As described above, in the aroma dispensing device 1 of the present invention, mixing proportions (aroma dispensing proportions) of the smells generated in the six element smell bottles 5a-5f are changed by opening and closing of the six solenoid valves 4a-4f. The aroma dispensing method will be described in detail below. As described above, opening and closing of the solenoid valves is performed by pulse operation at 20Hz, and one operation interval is 50msec. Accordingly, for example, if only the solenoid valve of the element smell bottle 5a is fully open and the other solenoid valves are closed, in other words, if only smell of the element smell bottle 5a is generated (, i.e., 100% of generated smell is smell of the element smell bottle 5a), a number of opening and closing per second of the solenoid valve 4a is 20, and those of the other solenoid valves are 0.

When a generated smell is intended to comprise 50% of a smell of the element smell bottle 4a and 50% of a smell of the element smell bottle 4b, a number of opening and closing per second of the solenoid valve 4a is 10 and that of the solenoid valve 4b is 10. More specifically, to generate a specific smell or aroma comprising 5% of a smell of the element smell bottle 5a, 10% of a smell of the element smell bottle 5b, 10% of a smell of the element smell bottle 5c, 15% of a smell of the element smell bottle 5d, 50% of a smell of the element smell bottle 5e, and 10% of a smell of the element smell bottle 5f, the solenoid valves 4a-4f are ON/OFFed 1, 2, 2, 3, 10 and 2 times, respectively. In this connection, the numbers of opening and closing are those per second. As described above, the recipe for the specific smell is the numbers of opening and closing per second of the solenoid valves, and no particular other information is needed. Accordingly, the recipe is simple and manageable.

What is important here is that the mixing proportions are equal to proportions of the numbers of opening and closing per second of the solenoid valves. For example, when a generated smell comprises 50% of a smell of the element smell bottle 5a and 50% of a smell of the element smell bottle 5b, the mixing proportions are 1 : 1 and the proportions of numbers of opening and closing per second of the solenoid valves are 10 :10, namely, 1:1. Further, when a generated smell comprises 5% of a smell of the element smell bottle 5a, 10% of a smell of the element smell bottle 5b, 10% of a smell of the element smell bottle 5c, 15% of a smell of the element smell bottle 5d, 50% of a smell of the element smell bottle 5e, and 10% of a smell of the element smell bottle 5f, the mixing proportions are 5 : 10 : 10 : 15 : 50 : 10 = 1: 2 ; 2 : 3 : 10: 2 and the proportions of numbers of opening and closing per second of the solenoid valves 4a-4f are 1 : 2 : 2 : 3 : 10 : 2.

Moreover, the mixing proportions of smells are equal also to the proportions of time periods of opening and closing per second of the solenoid valves. For example, when a generated smell comprises 50% of a smell of the element smell bottle 5a and 50% of a smell of the element smell bottle 5b, the mixing proportion are 1 : 1 and the proportions of opening and closing per second of the solenoid valves are 500msec : 500msec = 1 : 1. Further, when a generated smell comprises 5% of a smell of the element smell bottle 5a, 10% of a smell of the element smell bottle 5b, 10% of a smell of the element smell bottle 5c, 15% of a smell of the element smell bottle 5d, 50% of a smell of the element smell bottle 5e, and 10% of a smell of the element smell bottle 5f, the mixing proportions of smells are 5 : 10 : 10 : 15 : 50 : 10 = 1 : 2 : 2 : 3 : 10: 2 and the proportions of time periods of opening and closing per second of the solenoid valves 4a-4f are 50 : 100 : 100 : 150 : 500 : 100 (msec) = 1 : 2 : 2 : 3 : 10 : 2. Therefore, the aroma dispensing device of the present invention has a feature that the mixing proportions of smells and the proportions of numbers of opening and closing per second of the solenoid valves are equal to each other, and the mixing proportions of smells and the proportions of time periods of opening and closing per second of the solenoid valves are also equal to each other.

When a recipe for a specific smell is delivered over the internet, necessary information is only data on the element smell bottles (smell materials) and a number or time period of opening and closing of each of the solenoid valves. Accordingly, the information is easy-to-manage data for a distributor and can easily be incorporated in other information to be delivered. When a person has the aroma dispensing device of the present application and a recipe is downloaded via the internet in a room or the like, the system is less susceptible to noise because of simplicity of the data on the recipe and thus a problem of malfunction of the aroma dispensing device can be avoided.

Now, errors caused by the method for controlling the solenoid valves are described. In the aroma dispensing device 1 of the present invention, opening and closing of the solenoid valves is performed by pulse operation at 20Hz, and one interval of the operation is 50 msec. Accordingly, the minimum resolution performance in each of the element smell bottles 5a-5f is 5%. When a proportion of a certain smell material is intended to be 50%, i.e., the solenoid valve is so switched that a smell element of a certain element smell bottle is contained in a proportion of 50%, the operation of the solenoid valve is 10 times per second. This may be performed by 10 times of one (single) unit controlling, or may be performed by a continuous pulse controlling (n = 10). Further, the continuous pulse controlling may be performed divisionally by, for example, 3 times of two (double) unit controlling and 4 times of one (single) unit controlling. However, flow volumes are different according to the manners of controlling.

To attain 50% of the proportion of the smell material, if the pulse controlling is performed by only one (single) unit controlling, total flow volume is 36.5 x 10 = 365 Vu·msec. On the other hand, if the pulse controlling is performed by the continuous pulse controlling (N = 10), total flow rate volume is 36.5 + 50 x (10-1) = 486.5 Vu·msec. Further, if the pulse controlling is performed by 3 times of two (double) unit controlling and 4 times of one (single) unit controlling, total flow volume is 86.5 x 3 + 36.5 x 4 = 405.5 Vu·msec. Based on the continuous pulse controlling (n = 10), control error dependent on control algorithms is (486.5 - 365)/486.5 x 100 = 25 %. As described above, error can be caused according to the pulse controlling method. However, a problem can be avoided by specifying the controlling method.

A method for generating a smell or aroma using the aroma dispensing device of the present invention will be described. If it is supposed that smell sources (smell materials) are specified with respect to the first to sixth element smell bottles 5a-5f, proportions of the smell materials of the element smell bottles and a smell determined by the proportions may be stored preliminarily as a database. In this connection, the proportions mean proportions of individual smells of the element smell bottles based on the total. For example, a proportion of the smell of the element smell bottle 5a is 30% based on the total, and a proportion of the smell of the element smell bottle 5b is 20% based on the total.

Each of the element smell bottles may be bar-coded to prepare a database and managed therewith. In other words, when each of the element smell bottles is mounted on a holder, the bar-code labeled or printed on the element smell bottle is read to recognize what type of element smell bottle is provided. It is calculated using the bar-codes of the recognized element smell bottles how many types of smell can be synthesized or dispensed. By recording the results on any of various recording media, the database can be prepared. In this connection, the database may be housed in the aroma dispensing device body or may be housed in equipment for controlling the aroma dispensing device such as an external computer.

Data inputted in the database is, for example, such that in the case of a smell of rose, 20% of a smell of the element smell bottle 5a (4 times, 200 msec), 30% of a smell of the element smell bottle 5b (6 times, 300 msec) and 50% of a smell of the element smell bottle 5c (10 times, 500 msec). The numerical values in each of the parentheses are the above-described number and time period of opening and closing per second of the solenoid valve, respectively. Smalls which can be synthesized include a variety of smells such as those of apple, tomato, caramel, banana, almond, lavender, Japanese cypress and hiba arborvitae. These data can be stored as so-called recipes, and using the data, the same smells can be generated from other aroma dispensing devices.

When one aroma dispensing device and another aroma dispensing device have the same groups of element smell bottles, the same smell can be generated from these devices only with reference numbers of the element smell bottles and numbers of opening and closing per second of the solenoid valves. When one aroma dispensing device and another aroma dispensing device do not have the same groups of element smell bottles, the same smell cannot be generated from these devices only with reference numbers of the element smell bottles and numbers of opening and closing per second of the solenoid valves. In this case, however, the same smell can be generated from the devices by specifying the smell from a recipe, and anew preparing the recipe for generating the same smell with reference to the database. If no recipe for the same smell is stored in the database, the nearest smell thereto may be selected.

Next, modification according to temperature will be described. Smell materials are contained in the element smell bottles, and the smell materials include various types. In general, when introduced air (air blown against the smell material) has a higher temperature, the smell material tends to be volatilized easier to result in a higher concentration of the smell. On the other hand, when the air has a lower temperature, the smell material tends to be less easy-to-volatilize to result in a lower concentration of the smell. Accordingly, in the present invention, the amounts of air to be introduced into the element smell bottles are modified according to the temperature of the air. As described above, since the amounts of air to be introduced are determined by the numbers of opening and closing per second of the solenoid valves, the numbers of opening and closing are modified.

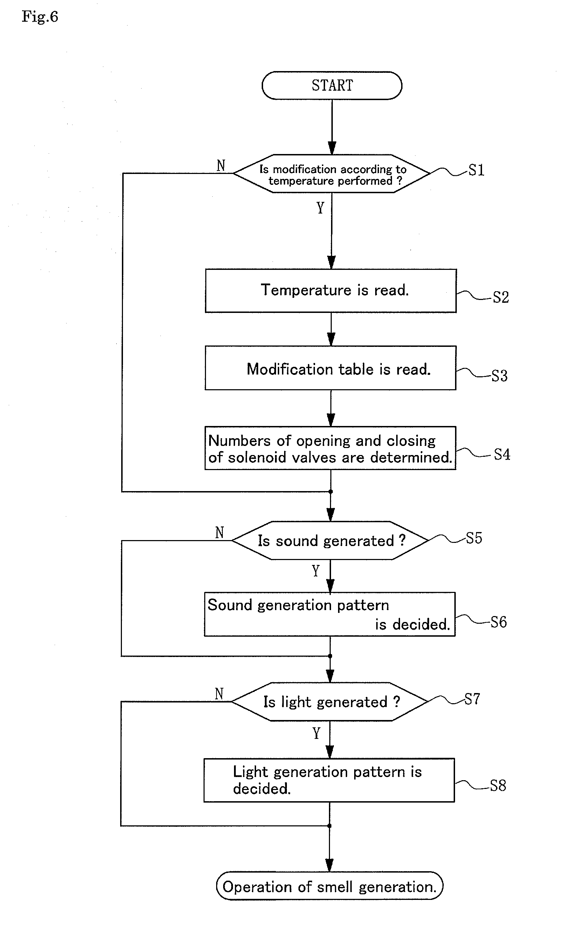

Fig.6 is a flowchart of modification according to temperature, followed by generation of a smell. In Fig.6, a flowchart of generation of a sound and generation of light is also incorporated. First, it is decided whether modification according to temperature is carried out or not (S 1). If not, the procedure advances to a step of whether generation of a sound is performed or not (S 5). When modification according to temperature is carried out, a temperature in the aroma dispensing device is read (S 2). As a temperature measuring means, a thermistor is used. The thermistor is disposed at a specific position in the aroma dispensing device. The temperature of the inside, including the element smell bottles, of the aroma dispensing device 1 is supposed to be that measured by the thermistor and uniform. Based on the measured temperature and a preliminarily prepared table for modification according to temperature, modification values are read (S 3). To the numbers of opening and closing which are allotted to respective solenoid valves for blending smells in specific proportions, the modification values are respectively added to determine final numbers of opening and closing of the solenoid valves (S 4).

Then, it is decided whether generation of a sound is performed or not (S 5). If not, the procedure advances to a step of whether generation of light is performed or not (S 7). When generation of a sound is performed, a sound generation pattern is decided (S 6). The decision of the sound generation pattern means decision of a music played in coincidence with generation of a smell. In conformity with the smell, a soothing music, an exhilarating music or the like may be played.

Then, it is decided whether generation of light is performed or not (S 7). If not, the procedure advances to smell generating operation. When light is generated, a light generation pattern is decided (S 8). The decision of the light generation pattern means deciding in what manner an LED is caused to emit light. For example, a triple-color (three primary colors: red, blue and green) LED may be caused to glow gradationally.

Table 1 shows an example of thermodependent modification values. For example, it is shown that with respect to the element smell bottle No.1, when the temperature is 20-25 °C, the number of opening and closing the solenoid valve is that for attaining a predetermined mixing proportion - 1.

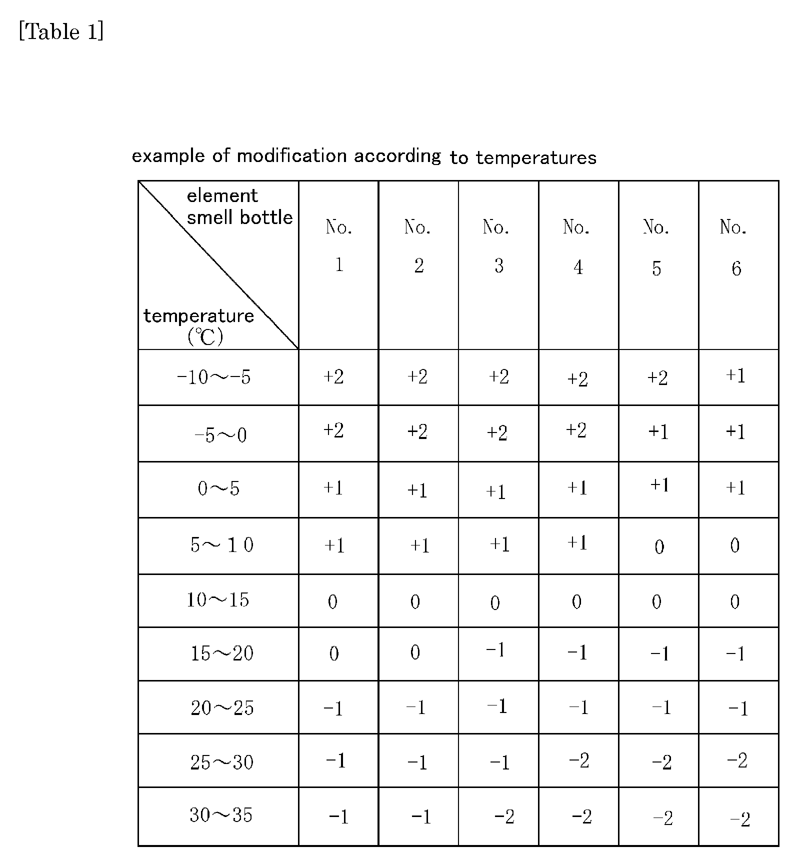

By effecting the modification according to temperature in this manner, if a working temperature of the aroma dispensing device is changed, quality of the generated smell can be maintained to some extent.

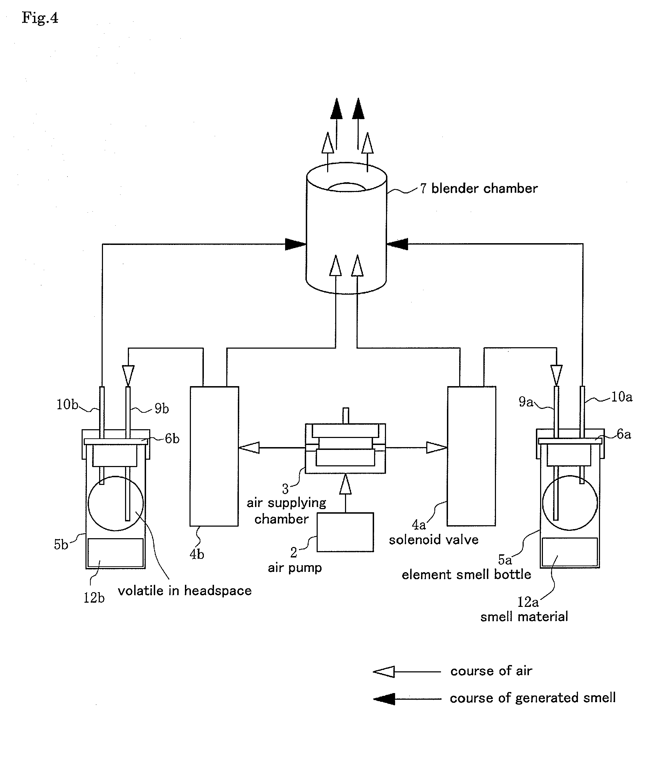

Fig.4 is a schematic diagram of a second embodiment of the aroma dispensing device according to the present invention. The aroma dispensing device of the second embodiment comprises an air pump 2, an air supplying chamber 3, six element smell bottles, six solenoid valves, and a blender chamber 7. The six element smell bottles are a first element smell bottle 5a, a second element smell bottle 5b, a third element smell bottle 5c, a fourth element smell bottle 5d, a fifth element smell bottle 5e, and a sixth element smell bottle 5f. The six solenoid valves are a first solenoid valve 4a, a second solenoid valve 4b, a third solenoid valve 4c, a fourth solenoid valve 4d, a fifth solenoid valve 4e, and a sixth solenoid valve 4f. Besides these, the device is provided with a thermistor for measuring a temperature, an LED which glows when the air pump is operated to generate a smell, a speaker which makes a sound also when a smell is generated, a control board for controlling the speaker and the LED, a control board for controlling the solenoid valves, an interface for internet delivery of smell by a computer, and the like, although they are not shown. In this connection, although only two element smell bottles and two solenoid valves are shown in Fig.4 as a matter of convenience, the six element smell bottoms and the six solenoid valves are provided in reality.

The second embodiment is different from the first embodiment in that the air returned to the air supplying chamber 3 when the solenoid valve is OFF in the first embodiment is not returned to the air supplying chamber but caused to flow directly into the blender chamber 7 in the second embodiment. For the connection between each of the solenoid valves and the blender chamber 7, a vinyl pipe 8 is used. Since other features are the same as in the first embodiment, explanation is omitted.

The advantage of causing the air to flow into the blender chamber 7 but not returning the air to the air supplying chamber 3 as described above resides in that the load on the air pump 2 can be reduced. In such a constitution that the air is returned to the air supplying chamber 3 when the solenoid valve is OFF, a larger load is imposed on the air pump because a pressure is exerted on the air supplying chamber 3 by the air pump 2. By causing the air to flow into the blender chamber 7, the load on the air pump 2 can be reduced and, in consequence, noise of the air pump can be suppressed. As compared with the first embodiment, air is discharged from the blender chamber 7 in a larger amount and, accordingly, diffusion of a specific smell is accelerated even if the smell has the same mixing proportions.

Also in the aroma dispensing device of the second embodiment, mixing proportions are equal to proportions of numbers of opening and closing per second of the solenoid valves, and the mixing proportions are equal to the proportions of time periods of opening and closing per second of the solenoid valves. Further, with respect also to modification according to temperature, the second embodiment is the same as the first embodiment.

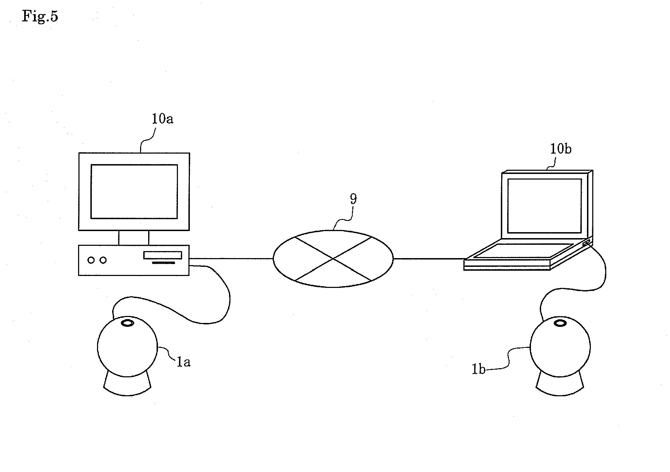

Fig.5 is a schematic diagram of a system for delivering a recipe for a smell via internet 9. Two computers 10a, 10b are connected via internet 9. To the computers 10a, 10b, aroma dispensing devices 1a, 1b are connected, respectively. The aroma dispensing devices 1a, 1b may be any of the first and second embodiments. For example, a case is considered where data on a specific smell is transmitted from the computer 10a to the computer 10b via internet 9. As described above, the data on the smell is numbers or time periods of opening and closing per second of the solenoid valves for the element smell bottles if the element smell bottles are the same between the aroma dispensing devices.

If the element smell bottles are not the same between the aroma dispensing device 1a and the aroma dispensing device 1b, data can be constructed as follows. Each of the element smell bottles is bar-coded and managed therewith, and a database can be prepared with respect to smells which can be generated. In other words, when each of the element smell bottles is mounted on a holder or the like, the bar-code labeled or printed on the element smell bottle is read to recognize what type of element smell bottle is provided. It is calculated using the bar-codes of the recognized element smell bottles how many types of smell can be synthesized or blended. By recording the results on any of various recording media, the database can be prepared. In this connection, the database may be stored in the aroma dispensing device body or may be stored in equipment for controlling the aroma dispensing device such as an external computer.

When a recipe for a smell is received, data for generating the same or similar smell is specified with reference to the database, and in accordance with the data, numbers or time periods of opening and closing per second of the solenoid valves are determined. Modification according to temperature may be effected as occasion requires. When the modification according to temperature is performed, it is necessary to attach temperature data to the recipe for the smell.

Data inputted in the database is, for example, such that in the case of a smell of rose, 20% of a smell of the element smell bottle 5a (4 times, 200 msec), 30% of a smell of the element smell bottle 5b (6 times, 300 msec) and 50% of a smell of the element smell bottle 5c (10 times, 500 msec). The numerical values in each of the parentheses are the number and time period of opening and closing per second of the solenoid valve, respectively. Smells which can be synthesized include a variety of smells such as those of apple, tomato, caramel, banana, almond, lavender, Japanese cypress and hiba arborvitae. The data can be stored as so-called recipes, and using the data, the same smells can be generated from other aroma dispensing devices. To enhance reliability of a dispensed smell, it is important to prescribe a temperature when dispensing is carried out. Using modification values relative to the temperature, the numbers of opening and closing per second of solenoid valves can be modified to enhance reliability of the dispensed smell.

To sum the above up, when one aroma dispensing device and another aroma dispensing device have the same groups of element smell bottles, the same smell can be generated from these devices by specifying reference numbers of the element smell bottles and the numbers or time periods of opening and closing per second of the solenoid valves. When one aroma dispensing device and another aroma dispensing device do not have the same groups of element smell bottles, the same smell cannot be generated from these devices only with the reference numbers of the element smell bottles and the numbers or time periods of opening and closing per second of the solenoid valves. In this case, however, it suffices to specify the smell to be dispensed from a recipe for the smell, and to anew prepare a recipe for generating the same smell with reference to the database. If no recipe for the same smell is stored in the database, the nearest smell thereto may be selected.

When a recipe for a specific smell is distributed over the internet, necessary information is only data on the element smell bottles (smell materials), and a number or time period of opening and closing per second of each of the solenoid valves, and as occasion requires, a temperature. The information is easy-to-manage data for a distributor and can easily be incorporated in other information to be transmitted. When a person has the aroma dispensing device of the present application and a recipe is downloaded via internet in a room or the like, the system is less susceptible to noise because of simplicity of the data on the received recipe and thus a problem of malfunction of the aroma dispensing device can be avoided.

The aroma dispensing device of the present invention is so constructed that when a smell is generated, a plurality of LEDs may be caused to glow and a sound may be made. By virtue of this, it is possible to enjoy illumination with the LEDs and to listen to music while taking the smell. It is, or course, significantly effective to have a body-healing smell while enjoying the illumination by means of the LEDs and listening to music.

### [Industrial Applicability]

The aroma dispensing device of the present invention comprises an air pump, element smell bottles, solenoid valves and a blender chamber and is so constructed as to be capable of highly precisely and simply dispensing smells of the element smell bottles, and accordingly, has industrial applicability as described below.

(1) In cooperation with visual sense:
(a) A smell befitting to a scene of a TV program or video is generated. In other words, the aroma dispensing device of the present invention is combined with a TV, movie equipment or DVD equipment to generate a smell befitting to a scene as occasion arises. It follows that for a viewer, realism is greatly enhanced. In this case, by superimposing a recipe for the smell on picture signals of a program to be broadcasted or image data of DVD, the smell can be generated automatically without any particular procedure for generating the smell.
(b) A smell is generated in conformity with a picture book. When a reader of the picture book looks at, for example, a picture of banana, a smell of banana is generated by the aroma dispensing device. In this case, it is possible that data on a recipe for the smell is preliminarily embedded in the picture of banana and the data is read by a pick-up device to generate the smell. Since the reader is able to have the real smell while looking at the picture, reader's understanding is deepened.
(2) In cooperation with acoustic sense:
(a) A smell is generated in conformity with a music which is being listened to or a song which is being sung. By generating the smell in harmony with an image of the music or song or melody, it is possible to enhance impression about the music which is being listened to or song which is being sung.
(b) An unpleasant smell is generated when a strange sound is detected. By generating the unpleasant smell when a suspicious individual attempts to intrude and makes an unusual sound, it is also possible to prevent intrusion of the suspicious individual.
(3) In cooperation with sense of touch:
A smell is generated by the aroma dispensing device of the present invention in combination with a massage tool. By having a massage while having a smell from which healing effect is expected, effect of the massage can be further increased. From the medical viewpoint, studies have been made on smells having a pain-relieving effect, smells having a sedative effect, and the like. Accordingly, it will actively be performed in the future to receive medical treatment in combination with smell therapy.
(4) In cooperation with internet:
A recipe for a smell is delivered through internet. When computers are connected via internet, it is possible to enjoy the same smell at any places in the world by delivering the recipe for the smell over internet. In regard to internet delivery of recipes for smells, various applications can be considered.
(5) In cooperation with cerebral nerve:
It is considered that the aroma dispensing device of the present invention is placed in one's own room and a smell for increasing appetite, for quitting smoking, for providing feeling of fullness, or for refreshing the spirits is generated as occasion arises. Further, a smell may be generated for training an animal.
(6) In cooperation with time:
The aroma dispensing device of the present invention is placed in one's own room and smells which are changed according to time zones are generated to set daily rhythm in good order.
The applicability is specifically exemplified above. However, these are given by way of example only. Besides these, various manners of utilization can be considered.

### [Brief Description of Drawings]

Fig.1 is a schematic diagram of the first embodiment of the aroma dispensing device of the present invention.
Figs.2(a) and 2(b) are a perspective overall view and a perspective view of an operative portion, respectively, of the aroma dispensing device in Fig.1.
Fig.3 is illustrative diagrams on controlling operations of the solenoid valve.
Fig.4 is a schematic diagram of the second embodiment of the aroma dispensing device according to the present invention.
Fig.5 is a schematic diagram of a system for delivering a recipe for a smell via internet using the aroma dispensing device in Fig.1 or 4.
Fig.6 is a flowchart of modification according to temperature in the aroma dispensing device in Fig.1.
Fig.7 is a schematic diagram of a conventional dispensing device (dispensing of one-component smell).
Fig.8 is a schematic diagram of a conventional dispensing device (dispensing of two-component smell).

### [Note on Reference Numbers]

1, 1a, 1b: aroma dispensing device
2: air pump
3: air supplying chamber
4a, 4b, 4c, 4d, 4e, 4f: solenoid valve
5a, 5b, 5c, 5d, 5e, 5f: element smell bottle
6a, 6b, 6c, 6d, 6e, 6f: lid of element smell bottle
7: blender chamber
8: vinyl pipe
9a, 9b, 9c, 9d, 9e, 9f: inlet pipe
10a, 10b, 10c, 10d, 10e, 10f: outlet pipe
12a, 12b, 12c, 12d, 12e, 12f: smell material
13a, 13b: computer
14: internet
17, 21: carrier gas bottle
18: sample bottle
19, 20: element smell gas bottle
27: solenoid valve
28, 29: solenoid valve for switching
30: sensor cell
30A, 30B: sensor
31: pump
32: frequency counter
33: computer
40: flowmeter
41: valved flowmeter
50: buffer tank

## Claims

1. An aroma dispensing method which comprises:
blowing air against a plurality of smell materials contained in a plurality of element smell bottles to volatilize the plurality of smell materials, there by generating a plurality of smells; and
blending the plurality of generated smells in specific mixing proportions;
wherein the mixing proportions are determined by numbers of opening per second of a plurality of solenoid valves (numbers of open condition per second of the solenoid valves: hereinafter referred to as "numbers of opening and closing per second of the plurality of solenoid valves") which are respectively connected to the element smell bottles; and
proportions of the numbers of opening and closing per second of the plurality of solenoid valves correspond to the mixing proportions.

2. An aroma dispensing method which comprises:
blowing air against a plurality of smell materials contained in a plurality of element smell bottles to volatilize the plurality of smell materials, there by generating a plurality of smells; and
blending the plurality of generated smells in specific mixing proportions;
wherein the mixing proportions are determined by (total) time periods of opening per second of a plurality of solenoid valves ((total) time periods of open condition per second of the solenoid valves: hereinafter referred to as "time periods of opening and closing per second of the plurality of solenoid valves") which are respectively connected to the element smell bottles; and
proportions of the time periods of opening and closing per second of the plurality of solenoid valves correspond to the mixing proportions.

3. An aroma dispensing method which comprises:
blowing air against a plurality of smell materials contained in a plurality of element smell bottles to volatilize the plurality of smell materials, there by generating a plurality of smells; and
blending the plurality of generated smells in specific mixing proportions;
wherein the mixing proportions are determined by a plurality of solenoid valves respectively connected to inlets of the plurality of element smell bottles; and
the air is sent to the element smell bottles when the solenoid valves are open, and the air is returned to an air supplying chamber for temporarily holding air which is connected to the solenoid valves when the solenoid valves are closed.

4. The aroma dispensing method according to claim 1 or 2, wherein the numbers or time periods of opening and closing per second of the plurality of solenoid valves are modified according to temperatures measured by a temperature measuring means disposed in an aroma dispensing device.

5. An aroma dispensing device which comprises:
a plurality of element smell bottles for containing smell materials and for holding smells generated by blowing air against the smell materials;
an air pump for supplying the air;
an air supplying chamber for temporarily holding the air supplied by the air pump;
a plurality of solenoid valves for controlling flow rates of the air in introduction of the air from the air supplying chamber to the plurality of element smell bottles; and
a blender chamber to which the air controlled by the plurality of solenoid valves is introduced for blending the smells generated in the plurality of element smell bottles;
wherein when the plurality of smells generated in the plurality of element smell bottles are blended in the blender chamber in specific mixing proportions, the mixing proportions are determined by numbers of opening and closing per second of the plurality of solenoid valves respectively connected to the plurality of element smell bottles; and
proportions of the numbers of opening and closing per second of the plurality of solenoid valves correspond to the mixing proportions.

6. An aroma dispensing device which comprises:
a plurality of element smell bottles for containing smell materials and for holding smells generated by blowing air against the smell materials;
an air pump for supplying the air;
an air supplying chamber for temporarily holding the air supplied by the air pump;
a plurality of solenoid valves for controlling flow rates of the air in introduction of the air from the air supplying chamber to the plurality of element smell bottles; and
a blender chamber to which the air controlled by the plurality of solenoid valves is introduced for blending the smells generated in the plurality of element smell bottles;
wherein when the plurality of smells generated in the plurality of element smell bottles are blended in the blender chamber in specific mixing proportions, the mixing proportions are determined by time periods of opening and closing per second of the plurality of solenoid valves respectively connected to the plurality of element smell bottles; and
proportions of the time periods of opening and closing per second of the plurality of solenoid valves correspond to the mixing proportions.

7. An aroma dispensing device which comprises:
a plurality of element smell bottles for containing smell materials and for holding smells generated by blowing air against the smell materials;
an air pump for supplying the air;
an air supplying chamber for temporarily holding the air supplied by the air pump;
a plurality of solenoid valves for controlling flow rates of the air in introduction of the air from the air supplying chamber to the plurality of element smell bottles; and
a blender chamber to which the air controlled by the plurality of solenoid valves is introduced for blending the smells generated in the plurality of element smell bottles;
wherein when the plurality of smells generated in the plurality of element smell bottles are blended in the blender chamber in specific mixing proportions, the mixing proportions are determined by the plurality of solenoid valves respectively connected to inlets of the plurality of element smell bottles; and
the air is sent to the element smell bottles when the solenoid valves are open, and the air is returned to the air supplying chamber for temporarily holding air which is connected to the solenoid valves when the solenoid valves are closed.

8. The aroma dispensing device according to claim 5 or 6, wherein the numbers or time periods of opening and closing per second of the plurality of solenoid valves are modified according to temperatures measured by a temperature measuring means disposed in the aroma dispensing device.

9. The aroma dispensing device according to any one of claims 5 to 8, wherein the plurality of element smell bottles are bar-coded and managed with the bar codes; and the aroma dispensing device has an arithmetic element for calculating smells which can be dispensed+ by means of the plurality of element smell bottles and a database for storing the results of the calculation by the arithmetic element.

10. The aroma dispensing device according to any one of claims 5 to 9, wherein the numbers or time periods of opening and closing per second of the plurality of solenoid valves are converted into electrical signals and thereby can be delivered over internet.

11. The aroma dispensing device according to any one of claims 5 to 10, wherein when the solenoid valves are open to generate a smell, a light emitting diode is caused to glow so as to effect cooperation between the smell and light of the light emitting diode.

12. The aroma dispensing device according to any one of claims 5 to 11, wherein when the solenoid valves are open to generate a smell, a sound is made so as to effect cooperation between the smell and the sound.
